# FASCICULE DE BREVET EUROPEEN

(11) **EP 1 594 498 B1**
(45) Date de publication et mention de la délivrance du brevet: **22.07.2009**
(21) Numéro de dépôt: 04712083.7
(22) Date de dépôt: 18.02.2004
(51) Int. Cl.: A61K 31/437, A61K 31/444, A61K 31/706, A61P 27/06, A61P 27/02

(54) **UTILISATION DE L'ÉTAZOLATE POUR LE TRAITEMENT DE PATHOLOGIES NEURODEGENERATIVES OCULAIRES**
VERWENDUNG VON ETAZOLAT ZUR BEHANDLUNG VON NEURODEGENERATIVEN AUGENERKRANKUNGEN
USE OF ETAZOLATE FOR THE TREATMENT OF DEGENERATIVE OCULAR PATHOLOGIES

(30) Priorité: 19.02.2003 FR 0302021
(43) Date de publication de la demande: 16.11.2005
(73) Titulaire: Exonhit Therapeutics S.A., 75017 Paris (FR)
(72) Inventeur: SCHWEIGHOFFER, Fabien, F-94120 Fontenay sous Bois (FR); RESINK, Annelies, 3001 Heverlee (BE); DESIRE, Laurent, F-75014 Paris (FR); ROUQUETTE, Magali, F-31000 Toulouse (FR)
(74) Mandataire: Becker, Philippe
(86) Numéro de dépôt international: PCT/FR2004/000366
(87) Numéro de publication internationale: WO 2004/073711

(56) Documents cités:
- EP-A- 0 583 821
- WO-A-00/15222
- WO-A-01/49321
- WO-A-01/58469
- WO-A-01/81348
- WO-A-95/28926
- WO-A-98/53856
- WO-A-02/051502
- WO-A-02/098878
- WO-A-03/016563
- WO-A-03/045949
- WO-A-03/099278
- WO-A-20/04011464
- WO-A-20/04024085
- US-A- 6 083 483
- US-A1- 2002 119 923
- US-A1- 2002 132 826
- US-A1- 2002 177 599
- DATABASE WPI Week 200315 19 décembre 2002 (2002-12-19), Derwent Publications Ltd., London, GB; AN 2003-156939 XP002294573 AOTSUKA T. ET AL.: "PDEIV inhibitors" & WO 02/100859 A (GRELAN PHARM CO LTD) 19 décembre 2002 (2002-12-19) -& EP 1 403 270 A (GRELAN PHARMACEUTICAL CO) 31 mars 2004 (2004-03-31)

## Description

La présente invention concerne le domaine de la biologie, de la génétique et de la médecine. Elle concerne notamment l'utilisation et l'étascolate pour la préparation d'une composition pharmaceutique destinée au traitement d'une maladie neurodégénérative oculaire, telle que définie dans les revendications. Elle décrit de nouvelles approches pour la détection, la caractérisation et/ou le traitement (ou la prise en charge) de pathologies neurodégénératives. L'invention décrit également des méthodes pour l'identification ou le criblage de composés actifs dans ces pathologies. L'invention décrit également les composés, gènes, cellules, plasmides ou compositions utiles pour la mise en oeuvre des méthodes ci-dessus. L'invention découle notamment de l'identification du rôle de la phosphodiestérase 4B et du récepteur périphérique aux benzodiazépines dans les pathologies neurodégénératives oculaires et décrit leur utilisation comme cible ou marqueur thérapeutique, diagnostique ou expérimental de ces désordres.

De nombreuses pathologies neurodégénératives ont été décrites comme ayant une composante ou un stade lié au phénomène d'apoptose ou mort cellulaire programmée. On peut citer aussi bien les pathologies neurodégénératives du système nerveux central (par exemple la Sclérose Latérale Amyotrophique - SLA-, la maladie de Parkinson ou la maladie d'Alzheimer), que les maladies dégénératives périphériques, notamment oculaires. Ces pathologies disposent actuellement de traitements symptomatiques, notamment de traitement des phénomènes inflammatoires associés, mais pas de traitement des causes réelles de ces désordres, en raison notamment de la complexité des mécanismes et voies métaboliques impliqués, et de la diversité des facteurs causatifs.

La demande WO03/045949 décrit des inhibiteurs de GSK3 utilisables dans les maladies neurodégénératives. Les demandes WO01/4932 et WO01/584 proposent l'utilisation de composés inhibiteurs du TNF pour traiter des pathologies neurologiques. La demande WO00/15222 propose d'utiliser des composés inhibiteurs de PDE5 dans le traitement des troubles de l'érection. La demande EP583821 propose des approches thérapeutiques pour le traitement de dérèglements du volume de fluides crâniens.

La demande internationale de brevet n° PCT/FR02/02861 déposée par la demanderesse décrit de nouvelles cibles moléculaires de la neurotoxicité, ainsi que de nouvelles approches thérapeutiques pour le traitement des pathologies neurodégénératives. Ces approches sont basées sur une modulation de l'activité ou de l'expression d'une phosphodiestérase de type 4, ainsi que sur des modifications de la régulation du récepteur périphérique aux benzodiazépines et du récepteur GABA(A).

La présente demande concerne maintenant de nouvelles stratégies thérapeutiques des maladies neurodégénératives oculaires. Ces stratégies sont basées sur une modulation d'une ou plusieurs voies métaboliques identifiées par les inventeurs, qui sont corrélées à l'apparition, au développement et à la progression de l'excitotoxicité et de l'apoptose dans les cellules nerveuses, et sont particulièrement pertinentes dans les maladies neurodégénératives oculaires.

Plus particulièrement, un répertoire des altérations de l'épissage de l'ARN dans le cerveau et la moelle épinière d'animaux modèles de l'SLA, âgés de 60 jours, a été identifié par criblage différentiel qualitatif selon la technique DATAS (décrite dans la demande n° WO99/46403). Ce répertoire a été construit à partir d'ARN extraits d'échantillons de cerveau et de moelle épinière, sans isolement préalable des neurones, afin de prendre en compte un maximum d'évènements d'épissages alternatifs liés au développement de la pathologie. Le répertoire ainsi produit contient plus de 200 séquences distinctes, impliquant des acteurs clefs du phénomène d'excitotoxicité, tels que les canaux potassiques et calcique. La spécificité des séquences qui constituent ce répertoire est attestée par le fait que la même analyse différentielle qualitative de l'expression génétique réalisée sur des animaux âgés de 90 jours aboutit à un répertoire différent, dont sont absents notamment les différents marqueurs de l'excitotoxicité. L'analyse des modifications d'épissage confirme que les évènements moléculaires sont différents selon le stade de la pathologie.

De manière particulièrement intéressante et inattendue, la réalisation de DATAS sur des ARN d'animaux contrôles et transgéniques âgés de 60 jours a permis d'isoler des fragments d'ADNc dérivés de l'ARNm de la phosphodiestérase 4B, de la protéine AKAP1 (A Kinase Anchoring Protein) et de la protéine GABA(A)RAPL1 (GABA(A) Receptor Associated Protein Like 1). La protéine PDE4B, capable d'hydrolyser l'AMPc, est impliquée dans la régulation de la concentration intracellulaire d'AMPc. La protéine AKAP1 ancre la sous-unité régulatrice de la protéine kinase A (activée par l'AMPc) à la membrane mitochondriale et régule l'activité du pore de transition mitochondrial en raison de son interaction avec le récepteur périphérique aux benzodiazépines (PBR).

La présente demande démontre ainsi l'implication des cascades de signalisation dépendantes de l'AMPc, de la régulation du PBR et de la signalisation dépendante du récepteur GABA(A) dans le développement des processus d'excitotoxicité et de mort neuronale.

Les résultats obtenus montrent plus précisément une expression plus prononcée de PDE4B dans les tissus nerveux pathologiques, liée à une modification structurale de l'ARN correspondant, notamment à la délétion d'une région dans la partie 3' non-codante. Ce résultat est tout à fait compatible avec la présence de séquences de déstabilisation des ARNm dans la séquence identifiée par DATAS. La délétion de ces séquences de déstabilisation de l'ARNm de la PDE4B, par épissage ou par utilisation de séquences de polyadénylation alternatives, peut aboutir à une stabilisation, donc à une augmentation de l'expression, de la partie codante de cet ARN. Cet événement se produit spécifiquement dans le cerveau des sujets pathologiques et non dans les sujets contrôles.

L'identification d'un fragment dérivé de AKAP1 démontre par ailleurs l'implication de cette protéine dans le développement des processus d'excitotoxicité et de mort neuronale. AKAP1 interagit avec la sous-unité régulatrice de la protéine kinase A et avec le récepteur périphérique aux benzodiazépines (PBR), qui participe à la régulation de l'ouverture du pore mitochondrial de transition, ouverture qui caractérise l'exécution de l'apoptose. Par conséquent l'invention suggère que AKAP1 régule l'intervention du PBR dans les phénomènes de morts cellulaires tels la mort neuronale.

L'identification d'un fragment dérivé de GABA(A)RAPL1 souligne une dérégulation de la signalisation dépendante du récepteur GABA(A). Cette observation est tout à fait compatible avec l'importance du neurotransmetteur comme inhibiteur de transmission synaptique, notamment par son interaction avec le récepteur GABA(A). Cette inhibition permet de protéger les neurones contre une excitation soutenue qui pourrait conduire à la mort neuronale par excitotoxicité. Nos travaux indiquent donc une altération de ce niveau de régulation.

La présente invention décrit donc trois événements moléculaires originaux caractérisés par une altération de l'expression de l'ARNm de la PDE4, de AKAP1 et de GABA(A)RAPL1 dans le cerveau de sujets pathologiques, et qui sont corrélés dans le temps avec le phénomène d'excitotoxicité et/ou de mort neuronale. Ces voies de signalisation, médiées par l'AMPc, régulant le PBR, et relayant les signaux dépendant du GABA, définissent de nouvelles stratégies pour le développement de thérapeutiques des maladies neurodégénératives, utilisables notamment à des phases précoces de leur évolution, et s'adressant aux véritables bases moléculaires de la pathologie et non aux symptômes ou composantes inflammatoires associées.

La possibilité d'agir sur l'une ou, de préférence, simultanément sur ces trois voies métaboliques conduirait ainsi à des traitements particulièrement efficaces des maladies neurodégénératives, notamment oculaires. En effet, il est connu que les neurodégénérences de la rétine (et notamment la perte des photorécepteurs) sont associées à des variations des taux rétinien de nucléotides cycliques (GMPc, AMPc). La modulation de ces taux pourrait donc être bénéfique dans le traitement de maladies neurodégénératives oculaires, notamment par le contrôle de l'excitoxicité.

L'invention concerne plus particulièrement l'utilisation de l'étazolate pour la préparation d'une composition pharmaceutique destinée au traitement d'une maladie neurodégénérative oculaire choisie parmi la dégénérescence maculaire liée à l'age, la rétinite pigmentaire et la rétinopathie.

L'invention concerne notamment l'utilisation de l'étazolate pour augmenter la survie neuronale chez les patients atteints de ladite maladie neurodégénérative oculaire.

L'invention concerne également l'utilisation de l'étazolate pour inhiber ou réduire la mort neuronale par excitotoxicité chez les patients atteints de ladite maladie neurodégénérative oculaire.

L'invention décrit également le développement de tests, kits ou procédés de détection, dépistage ou diagnostic in vitro de ces pathologies, basés sur une détermination de la présence d'une dérégulation ou d'une altération dans un gène, un messager ou une protéine PDE4 ou, AKAP1 ou encore GABA(A)RAPL1 chez un sujet. L'invention décrit également des outils pour la mise en oeuvre de tels tests, notamment des sondes, amorces, cellules, réactifs, etc.

L'invention décrit également des tests ou procédés pour cribler des molécules candidates pour le traitement des maladies neurodégénératives, comprenant la détermination de la capacité des molécules à lier le récepteur PBR, AKAP1, GABA(A)RAPL1, le récepteur GAB(A) et/ou la PDE4.

### Thérapie

La présente invention décrit donc, de manière générale, l'utilisation d'inhibiteurs de PDE4 et/ou de ligands de PBR et de GABA(A) pour le traitement de maladies neurodégénératives oculaires.

L'utilisation d'inhibiteurs de PDE4 n'a jamais été envisagée pour améliorer la viabilité neuronale et plus particulièrement leur protection contre l'excitotoxicité. Les inhibiteurs de PDE4, développés pour inhiber les phénomènes inflammatoires, ont été suggérés comme potentiellement utiles dans des pathologies neurodégénératives centrales comme la maladie d'Alzheimer. Cette suggestion s'appuie sur la volonté de réduire l' inflammation observée dans le cerveau au cours des processus neurodégénératifs et nullement sur un rationnel visant à inhiber directement la mort neuronale. En outre, cette suggestion ne concerne nullement les maladies périphériques, notamment oculaires.

La présente invention montre l'existence d'événements d'épissage ou de sites de polyadénylation alternatifs affectant les gènes de la PDE4 et de AKAP1 et de GABA(A)RAPL1, associés au développement de l'excitotoxicité neuronale, et fournit la base moléculaire qui justifie l'utilisation d'inhibiteurs de PDE4 et/ou de ligands du PBR et de récepteur GABA(A) pour le traitement des maladies neurodégénératives oculaires et plus généralement pour améliorer la viabilité neuronale lors des phénomènes d'excitotoxicité, en particulier dès les phases précoces de ces pathologies.

L'invention concerne plus particulièrement l'utilisation de l'étazolate pour la préparation d'une composition pharmaceutique destinée au traitement d'une maladie neurodégénérative oculaire choisie parmi la dégénérescence maculaire liée à l'age, la rétinite pigmentaire et la rétinopathie.

Dans le contexte de l'invention, le terme « traitement » désigne le traitement préventif, curatif, palliatif, ainsi que la prise en charge des patients (réduction de la souffrance, réduction du déficit fonctionnel, amélioration de la durée de vie, ralentissement de la progression de la maladie, amélioration de la survie des neurones, protection des neurones contre l'excitotoxicité ou l'apoptose, etc.). Le traitement peut en outre être réalisé en combinaison avec d'autres agents ou traitements, notamment intervenant sur les événements tardifs de la pathologie, tels que des inhibiteurs de caspases ou autres composés actifs.

L'étazolate est un composé de formule suivante:

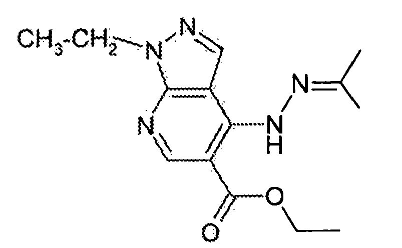

L'étazolate peut être formulé et administré de différentes façons. L'administration peut être réalisée par toute voie connue de l'homme du métier, de préférence par voie orale ou par injection, systémique, locale ou loco-régionale. L'injection est typiquement réalisée par voie intra-oculaire, rétro-oculaire, intra-péritonéale, intra-cérébro-ventriculaire, intra-rachidienne, intraveineuse, intra-artérielle, sous-cutanée ou intra-musculaire. L'administration par voie orale, systémique ou rétro- ou intra-oculaire est préférée. Les doses administrées peuvent être adaptées par l'homme de l'art. Typiquement, de 0,01 mg à 100 mg / kg environ sont administrés, pour des composés inhibiteurs de nature chimique. Il est entendu que des administrations répétées peuvent être réalisées, éventuellement en combinaison avec d'autres agents actifs ou tout véhicule ou excipient acceptable sur le plan pharmaceutique (ex., tampons, solutions saline, isotonique, en présence d'agents stabilisants, etc.).

Le véhicule ou excipient acceptable sur le plan pharmaceutique peut être choisi parmi des solutés tampons, solvants, liants, stabilisants, émulsifiants, etc. Des solutés tampons ou diluant sont notamment le phosphate de calcium, sulfate de calcium, lactose, cellulose, kaolin, mannitol, chlorure de sodium, amidon, sucre en poudre et hydroxy propyl methyl cellulose (HPMC) (pour libération retard). Des liants sont par exemple l'amidon, la gélatine et des solutés de remplissage comme le sucrose, glucose, dextrose, lactose, etc. Des gommes naturelles ou synthétiques peuvent aussi être utilisées, comme notamment l'alginate, la carboxymethylcellulose, la méthylcellulose, la polyvinyl pyrrolidone, etc. D'autres excipients sont par exemple la cellulose et du stéarate de magnésium. Des agents stabilisants peuvent être incorporés aux formulations, comme par exemple des polysaccharides (acacia, agar, acide alginique, gomme guar et tragacanth, la chitine ou ses dérivés et des éthers de cellulose. Des solvants ou solutés sont par exemple la solution Ringer, l'eau, l'eau distillée, des tampons phosphates, des solutions salines phosphatées, et autres fluides conventionnels. Des formulations de type collyre, gouttes, etc. peuvent également être réalisées.

L'invention est utilisable chez les mammifères, notamment chez l'être humain. Les résultats présentés dans les exemples illustrent l'efficacité de l'invention pour améliorer la viabilité de neurones placés en conditions d'excitotoxicité, notamment sur cultures de rétines, et décrivent les résultats d'administrations chez l'homme.

D'autres aspects et avantages de la présente invention apparaîtront à la lecture des exemples qui suivent, qui doivent être considérés comme illustratifs.

### LEGENDE DES FIGURES

Figure 1: PCR semi-quantitative de PDE4B à partir d'échantillons de cerveau (1A) et de muscle (1 B).
Figure 2: Effet neuroprotecteur de l'étazolate sur la toxicité induite par NMDA/serine sur les cellules granulaires du cervelet.
Figure 3: Effet neuroprotecteur de l'étazolate sur la toxicité induite par le kainate sur les cellules granulaires du cervelet.
Figure 4: Effet neuroprotecteur de l'étazolate sur la toxicité induite par NMDA/serine sur les neurones corticaux.
Figure 5: Effet neuroprotecteur de l'étazolate sur la toxicité induite par le kainate sur les neurones corticaux.
Figure 6 : Effet neuroprotecteur de l'étazolate sur la toxicité induite par NMDA/sérine sur les cellules de moelle épinière ventrale
Figure 7 : Effet neuroprotecteur de l'étazolate sur la rétine.

### EXEMPLES

### Exemple 1 : Identification de la PDE4, de AKAP1 et/ou de GABA(A)RAPL1 comme cibles moléculaires de l'excitotoxicité

L'analyse qualitative différentielle a été effectuée à partir d'ARN poly adénylés (poly A+) extraits d'échantillons de cerveaux d'animaux correspondant aux différents stades, sans isolement préalable des neurones afin de prendre en compte un maximum d'événements d'épissages alternatifs liés au développement de la pathologie.

Les ARN poly A+ sont préparés selon des techniques connues de l'homme de métier. Il peut s'agir en particulier d'un traitement au moyen d'agents chaotropiques tels que le thiocyanate de guanidium suivi d'une extraction des ARN totaux au moyen de solvants (phénol, chloroforme par exemple). De telles méthodes sont bien connues de l'homme du métier (voir Maniatis et al., Chomczynsli et al., Anal. Biochem. 162 (1987) 156), et peuvent être aisément pratiquées en utilisant des kits disponibles dans le commerce. A partir de ces ARN totaux, les ARN poly A+ sont préparés selon des méthodes classiques connues de l'homme de métier et proposées par des kits commerciaux.

Ces ARN poly A+ servent de matrice à des réactions de transcription inverse à l'aide de reverse transcriptase. Avantageusement sont utilisées des reverse transcriptases dépourvues d'activité RNase H qui permettent d'obtenir des premiers brins d'ADN complémentaire de tailles supérieures à ceux obtenues avec des reverse transcriptases classiques. De telles préparations de reverse transcriptases sans activité RNase H sont disponibles commercialement.

Pour chaque point de la cinétique de développement de la pathologie (30 jours, 60 jours et 90 jours) les ARN poly A+ ainsi que les ADNc simple brins sont préparés à partir des animaux transgéniques (T) et des animaux contrôles syngéniques (C).

Conformément à la technique DATAS, pour chaque point de la cinétique sont réalisées des hybridations d'ARNm (C) avec des ADNc (T) et des hybridations réciproques d'ARNm (T) avec des ADNc (C).

Ces hétéroduplex ARNm/ADNc sont ensuite purifiés selon les protocoles de la technique DATAS.

Les séquences d'ARN non appariées avec un ADN complémentaire sont libérées de ces hétéroduplex sous l'action de la RNase H, cette enzyme dégradant les séquences d'ARN appariées. Ces séquences non appariées représentent les différences qualitatives qui existent entre des ARN par ailleurs homologues entre eux. Ces différences qualitatives peuvent être localisées n'importe où sur la séquence des ARN, aussi bien en 5', 3' ou à l'intérieur de la séquence et notamment dans la séquence codante. Selon leur localisation, ces séquences peuvent être non seulement des modifications d'épissage mais également des conséquences de translocations ou de délétions.

Les séquences d'ARN représentant les différences qualitatives sont ensuite clonées selon les techniques connues de l'homme de métier et notamment celles décrites dans le brevet de la technique DATAS.

Ces séquences sont regroupées au sein de banques de cDNA qui constituent des banques qualitatives différentielles. Une de ces banques contient les exons et les introns spécifiques de la situation saine ; les autres banques contiennent les évènements d'épissage caractéristiques des conditions pathologiques.

L'expression différentielle des clones a été vérifiée par hybridation avec des sondes obtenues par reverse-transcription à partir d'ARN messagers extraits des différentes situations étudiées. Les clones hybridant de façon différentielle ont été retenus pour analyse ultérieure. Les séquences identifiées par DATAS correspondent à des introns et/ou à des exons exprimées de façon différentielle par épissage entre les situations pathologiques et la situation saine. Ces événements d'épissage peuvent être spécifiques d'une étape donnée du développement de la pathologie ou caractéristiques de l'état sain.

La comparaison de ces séquences avec les banques de données permet de classifier les informations obtenues et de proposer une sélection raisonnée des séquences selon leur intérêt diagnostique ou thérapeutique.

La réalisation de DATAS sur des ARN d'animaux contrôles et transgéniques âgés de 60 jours a permis d'isoler un fragment d'ADNc dérivé de l'ARNm de la phosphodiestérase 4B. Ce fragment correspond à un fragment d'exon spécifiquement présent dans les animaux contrôles et donc spécifiquement délété dans les animaux transgéniques pour SOD1 G93A au stade 60 jours. Ce fragment recouvre les nucléotides 377 à 486 référencés à partir du codon stop de la PDE4B de souris (séquence accessible dans GenBank, n°AF208023). Cette séquence comprend 2912 bases, le fragment délété correspondant aux bases 2760 à 2869. Cette région est non codante et est exprimée différentiellement entre les animaux contrôles et les animaux transgéniques, du fait de l'utilisation alternative d'un exon 3' non codant ou du fait de l'utilisation de deux sites de polyadénylation alternatifs.

La réalisation de DATAS sur des ARN d'animaux contrôles et transgéniques âgés de 60 jours a également permis d'isoler un fragment d'ADNc dérivé de l'ARNm de AKAP1. Ce fragment correspond à un fragment d'exon spécifiquement présent dans les animaux contrôles et donc spécifiquement délété dans les animaux transgéniques pour SOD1 G93A au stade 60 jours. Ce fragment est homologue aux nucléotides 1794 à 2322 de la séquence référencée dans GenBank sous le n°NM_009648 . Cette région est codante et est exprimée différentiellement entre les animaux contrôles et les animaux transgéniques, du fait d'un épissage alternatif.

La réalisation de DATAS sur des ARN d'animaux contrôles et transgéniques âgés de 60 jours a également permis d'isoler un fragment d'ADNc dérivé de l'ARNm de GABA(A)RAPL1. Ce fragment correspond à un fragment d'exon spécifiquement présent dans les animaux contrôles et donc spécifiquement délété dans les animaux transgéniques pour SOD1 G93A au stade 60 jours. Ce fragment est homologue aux nucléotides 1055 à 1461 de la séquence référencée dans GenBank sous le n°BC024706. Cette région est dérive de la région 3' non-codante et est exprimée différentiellement entre les animaux contrôles et les animaux transgéniques.

### Exemple 2 : Expériences de RT-PCR : Confirmation de l'expression différentielle :

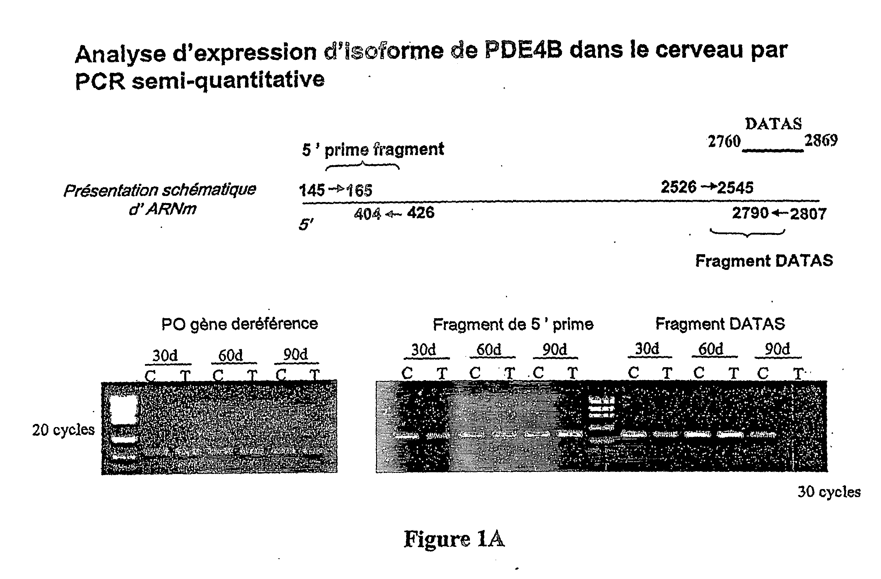

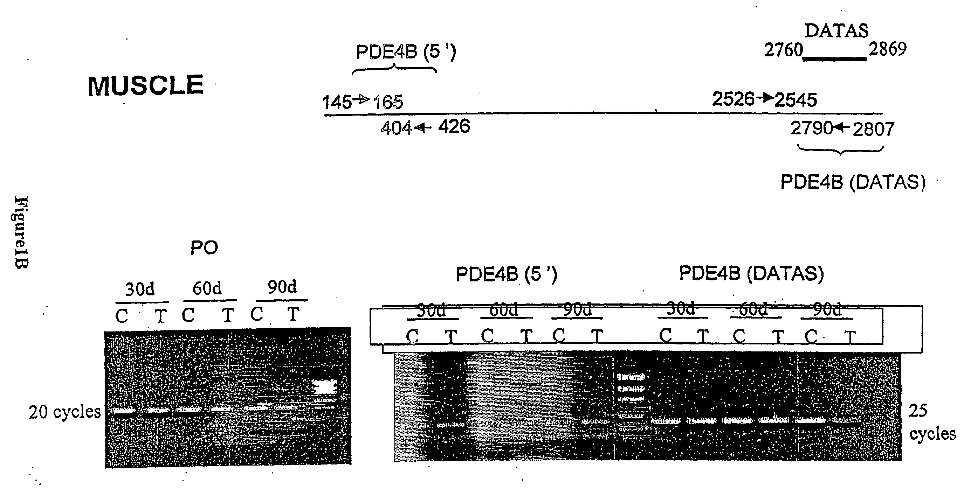

L'expression différentielle de la PDE4B dans une situation de stress neuronal, par rapport à une situation de référence, a été vérifiée par des expériences de RT PCR présentées sur la figure 1.

Ces expériences ont été réalisées selon des techniques bien connues de l'homme de métier et ont permis de suivre les expressions de deux régions distinctes de l'ARNm de la PDE4B. Une de ces régions recouvre le codon d'initiation de cet ARNm (PDE4B 5'), l'autre recouvre en partie le fragment identifié selon la technique DATAS (PDE4B DATAS). Les localisations des amorces de PCR utilisées sont indiquées sur la figure 1.

L'ARN PO correspond à un ARN ribosomal utilisé comme contrôle interne destiné à vérifier que la même quantité d'ARN est utilisée pour chaque point expérimental. Les analyses ont été réalisées à partir d'ARN extraits d'animaux contrôles (C) et transgéniques (T) âgés de 30, 60 et 90 jours, c'est à dire avant l'apparition des symptômes pathologiques.

Les ARN totaux du cerveau des souris contrôle ou SOD1 G93A âgées 30, 60 et 90 jours sont transcrits en ADNc utilisant le protocole standard de Superscript^{™} (Invitrogen). Pour les PCR semi-quantitatives les produits de la réaction de reverse transcription sont dilués 10 fois. Les amorces spécifiques du fragment DATAS correspondent pour le sens aux nucléotides 2526-2545 (5' GCC AGG CCG TGA AGC AAA TA 3' ; SEQ ID NO: 1), et pour l'anti-sens aux 2790-2807 (5' TCA AAG ACG CGA AAA CAT 3'; SEQ ID NO : 2) et pour le fragment plus en 3 prime les amorces correspondent pour le sens aux nucléotides 145-165 (5' CCG CGT CAG TGC CTT TGC TAT 3'; SEQ ID NO : 3), et pour l'anti-sens aux 426-404 (5' CGC TGT CGG ATG CTT TTA TTC AC 3'; SEQ ID NO : 4). Comme gène de référence le gène P0 est utilisé et amplifié par les amorces, sens : 5' TCG CTT TCT GGA GGG TGT C 3' (SEQ ID NO : 5) et anti-sens : CCG CAG GGG CAG CAG TGG 3' (SEQ ID NO :6).

L'amplification est effectuée par 30 cycles de PCR suivants :
- 30 secondes à 94°C
- une minute à 57°C
- 30 secondes à 72°C, suivi par un cycle de 2 minutes à 72°C

Les différents produits de PCR sont mis sur un gel d'agarose de 1.5 %. L'expérience est répétée trois fois avec deux réactions de reverse transcription différentes.

La figure 1 présente les résultats obtenus à partir d'ARN extraits des cerveaux ou des muscles des animaux.

Alors que la même quantité d'ADNc est amplifiée à partir de l'ARN de PO dans tous les échantillons, de variations sont observées pour l'ARNm de la PDE4B : les variations les plus significatives sont détectées chez les animaux âgés de 90 jours : alors qu'une augmentation du niveau d'expression du fragment PDE4 5' est observée dans le cerveau des animaux transgéniques, une très forte diminution de l'expression de PDE4B (DATAS) est observée dans le cerveau des animaux transgéniques.

Ce résultat établit une corrélation entre la diminution de l'expression d'un fragment 3' non codant de l'ARNm de la PDE4B et l'augmentation de l'expression de la partie 5' codante de ce même messager. Ce résultat est tout à fait compatible avec la présence de séquences de déstabilisation des ARNm dans la séquence identifiée par DATAS et démontre la corrélation entre l'expression de PDE4B et le phénomène d'excitotoxicité.

### Exemple 3: Inhibition de l'excitotoxicité par des ligands de PBR inhibiteurs de PDE4 (référence)

Pour cet exemple, des neurones granulaires du cervelet, des neurones corticaux ainsi que des cellules de moelle épinière ventrale de rat ont été mis en culture selon les techniques connues de l'homme de métier.

### Culture primaire des cellules granulaires de cervelet:

Les rats Wistar âgés de sept jours sont décapités et leurs cervelets sont disséqués. Après avoir enlevé les méninges, le tissu est coupé en petits morceaux et trypsinisé pendant 15 minutes à 37°C. Les cellules sont dissociées par trituration et mises en cultures à une densité 300.000 cellules par cm² dans du milieu basal Eagle supplémenté avec 10% du sérum de veau foetal et 2 mM glutamine. Le lendemain 10 µM ARA-C, un anti-mitotique, est ajouté pour empêcher la prolifération des cellules gliales. Les cellules sont traitées le jour 9 de cultures avec le composé inhibiteur étazolate, trois heures avant l'addition des toxiques, 50 µM kainate ou 100 µM N-methyl-D-aspartate en présence de 10 µM D-sérine. Le 8-bromo-cAMP est ajouté juste avant les toxiques. Tous les traitements sont effectués au minimum en double et dans au moins deux cultures différentes. Après une incubation de six heures la toxicité est mesurée par un test MTT. Les résultats, normalisés à la moyenne du non-traité, sont statistiquement analysés par le test de Wilcoxon. La valeur significative est déterminée à p inférieur ou égal à 0.05.

### Cultures primaires des cellules corticales :

Des embryons de rat Wistar, âgés de 16 jours, sont prélevés et les cortex sont disséqués. Après la trypsination à 37°C pendant 25 minutes, les cellules sont dissociées par trituration. Les cellules sont ensemencées dans du milieu essentiel minimum, supplémenté avec 10% de sérum de cheval et 10% de sérum de veau foetal et 2 mM glutamine, à une densité de 300.000 cellules par cm². Après 4 jours en culture la moitié du milieu est changée avec du milieu essentiel minimum supplémenté avec 5% de sérum de cheval et 2 mM glutamine. Le même jour, 10 µM de 5-fluoro-2-deoxyuridine, un anti-mitotique, est ajouté. Après sept et onze jours de culture, la moitié du milieu est changée par du milieu conditionné. Le milieu conditionné est composé de MEM contenant 5 % de sérum de cheval et 2 mM glutamine ; ce milieu est passé sur un tapis d'astrocytes corticales pendant une nuit avant son utilisation. A jour 14, les cellules sont traitées avec le composé inhibiteur étazolate, une heure avant l'addition des toxiques, 50 µM kainate ou 20 µM N-methyl-D-aspartate en présence de 10 µM D-sérine. Tous les traitements sont effectués au minimum en double et dans au moins deux cultures différentes. Après une incubation de six heures la toxicité est mesurée par un test MTT. Les résultats, normalisés à la moyenne du non-traité, sont statistiquement analysés par le test de Wilcoxon. La valeur significative est déterminée à p inférieur ou égal à 0.05.

### Cultures primaires de cellules de moelle épinière ventrale

Les cellules sont isolées à partir d'embryons de rat Wistar âgés de 14 jours. A leur arrivée, les rates gestantes sont sacrifiées par du dioxyde de carbone. Le chapelet d'embryons est prélevé et mis dans une boîte contenant du PBS. La moelle épinière de chaque embryon est disséquée et la corde ventrale est séparée des cordes dorsales. Les cordes ventrales sont ensuite trypsinisées à 37°C pendant 20 min. L'effet de la trypsine est arrêté par l'addition d'un milieu composé de milieu Leibovitz 15, 20% de sérum de cheval, supplément N2 (1X), 20% de glucose (3.2mg/ml), 7.5% de bicarbonate (1.8mg/ml) et de L-glutamine (2mM). Les cellules sont dissociées par trituration. Les amas tissulaires sont enlevés et les cellules dissociées sont ensuite quantifiées par coloration au bleu de trypan. Les cellules ensemencées à une densité de 250 000 cellules/cm² dans un milieu composé de milieu neurobasal, de sérum de cheval (2%), de supplément B27 (1X), et de glutamine (2mM). Après 3 jours de culture *in vitro,* un agent anti-mitotique, l'ARA-C (5µM), est ajouté aux cellules afin d'inhiber la production de cellules gliales. Les cellules sont mises en culture à 37°C dans un incubateur humidifié (5% CO2) pour 9 jours. Après 9 jours de culture, les cellules sont traitées avec le composé inhibiteur : l'étazolate, 3 heures et 1 heure respectivement avant l'ajout de toxiques : 50µM kainate ou 100µM N-methyl-D-aspartate (NMDA) en présence de 10µM D-sérine. Tous les traitements sont effectués au minimum en double et dans au moins deux cultures différentes. Après 3 heures d'incubation avec NMDA/D-serine comme toxique et 1 heure d'incubation avec kainate, la toxicité est révélée par test MTT. Les résultats sont normalisés à la moyenne des contrôles non traités et analysés statistiquement par un test de Wilcoxon avec p inférieure à 0.05.

### MTT:

La toxicité est mesurée en utilisant le test MTT. Après l'incubation avec les composés, du MTT est ajouté à une concentration finale de 0.5 mg/ml par puits. Les plaques sont ensuite incubées pendant 30 minutes à 37, °C dans le noir. Le milieu est aspiré et les cristaux sont resuspendus dans 500 µl de DMSO (dimethylsulfoxyde). L'absorbance à 550 nm est lue et le pourcentage de viabilité est calculé.

### Résultats :

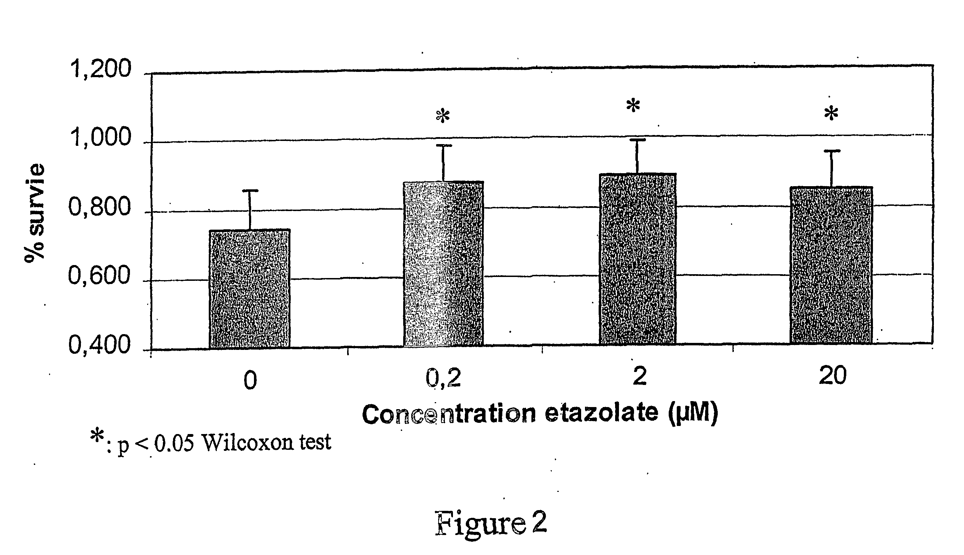

Les résultats obtenus sont présentés sur les figures 2-6. Ces résultats illustrent l'effet protecteur des composés de l'invention sur la survie neuronale. Lors du co-traitement des neurones par un inhibiteur de l'invention, un effet protecteur dose-dépendent est observé dans les deux modes d'induction de l'excitotoxicité (NMDA/Serine et kainate).

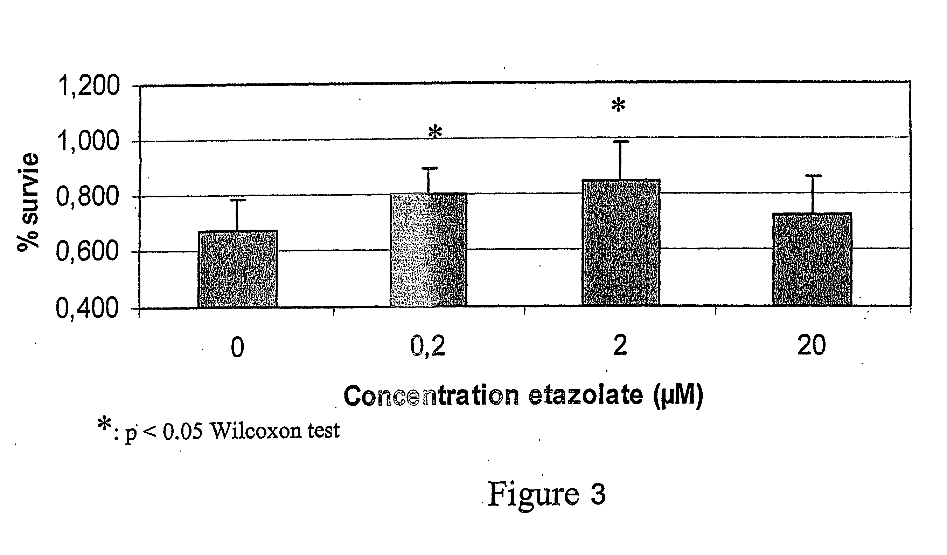

Les figures 2 et 3 présentent des résultats obtenus à l'aide de l'étazolate sur les cellules granulaires du cervelet. Les résultats présentés montrent que l'étazolate permet d'atteindre sur ces cellules un effet protecteur de 60% dans le cas du traitement NMDA/Ser, et de 57% dans le cas de la toxicité induite par le kainate.

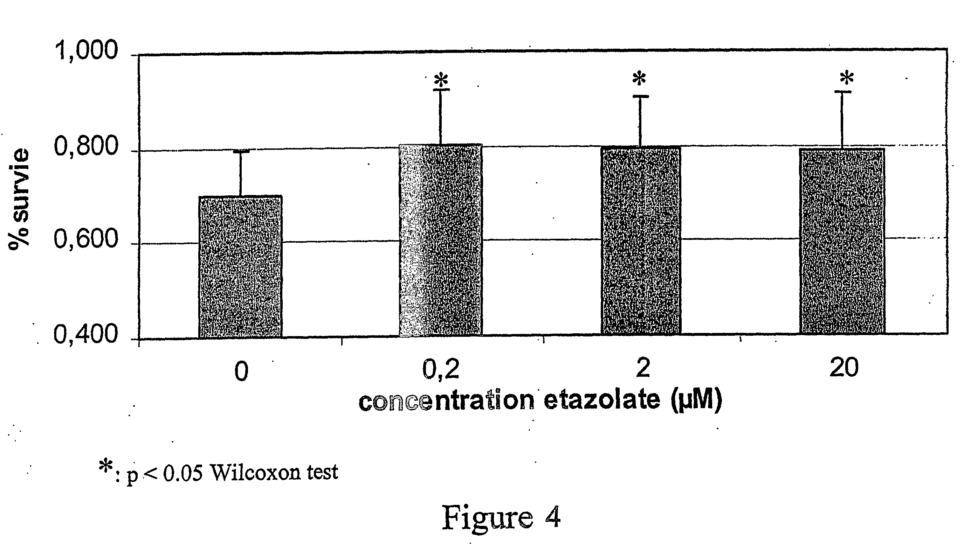

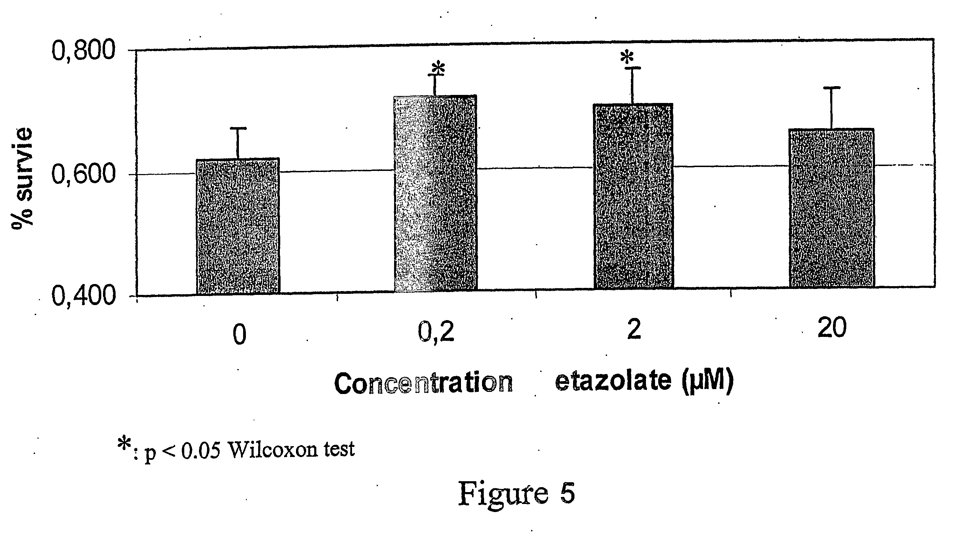

Les figures 4 et 5 présentent des résultats obtenus à l'aide de l'étazolate sur les neurones corticaux. Les résultats présentés montrent que l'étazolate permet d'atteindre sur ces cellules un effet protecteur de 33% dans le cas du traitement NMDA/Ser, et de 25% dans le cas de la toxicité induite par le kainate.

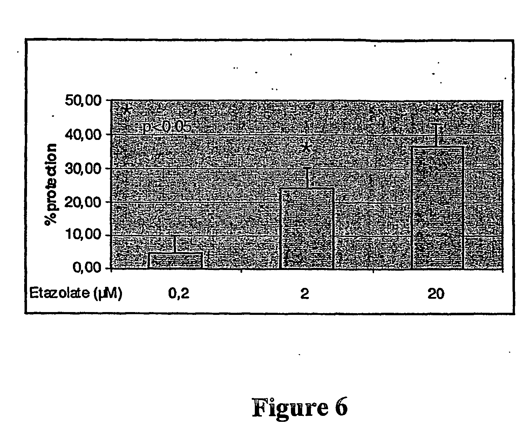

La figure 6 présente les résultats obtenus avec l'étazolate sur les cellules de moelle épinière ventrale. Ces résultats montrent que l'étazolate permet d'atteindre sur ces cellules un effet protecteur de 36% dans le cas du traitement NMDA/Ser.

La présente invention documente donc non seulement l'implication de la PDE4B, de PBR et des récepteurs GABA(A) dans les mécanismes d'excitotoxicité, mais également la capacité d'inhibiteurs et de ligands à préserver la viabilité neuronale lors de stress liés à l'excitotoxicité.

### Exemple 4 : Expériences in vitro utilisant la culture de rétine

Cet exemple décrit les effets d'un inhibiteur de PDE4 sur la rétine de souris rd1, souris caractérisée par la perte de cellules visuelles dès les deux premières semaines après la naissance.

Pour chaque concentration d'étazolate (0,02 µM, 0,2 µM, 2 µM et 20 µM), six explants de rétine de souris rd1 sont mis en culture pendant 21 jours. La culture commence au jour postnatal 7 et s'effectue dans un milieu en absence de sérum foetal bovin mais contenant l'étazolate à la concentration donnée. Six explants de rétine des mêmes animaux sont utilisés comme contrôles des explants traités. Dans ce cas, les explants sont cultivés dans du milieu de culture seul ou dans du milieu de culture contenant la solution dans laquelle l'étazolate est diluée. Le milieu de culture des deux conditions (contrôle et traitement) est changé tous les deux jours. Après 21 jours de culture, les explants sont fixés, sectionnés et marqués pour un examen histopathologique.

Les explants de souris rd1 non traités (jour postnatal 28) ont perdu la majorité de leurs photorécepteurs en culture, et la présence d'une augmentation des photorécepteurs est alors détectée en comptant les photorécepteurs restants.

### Résultats

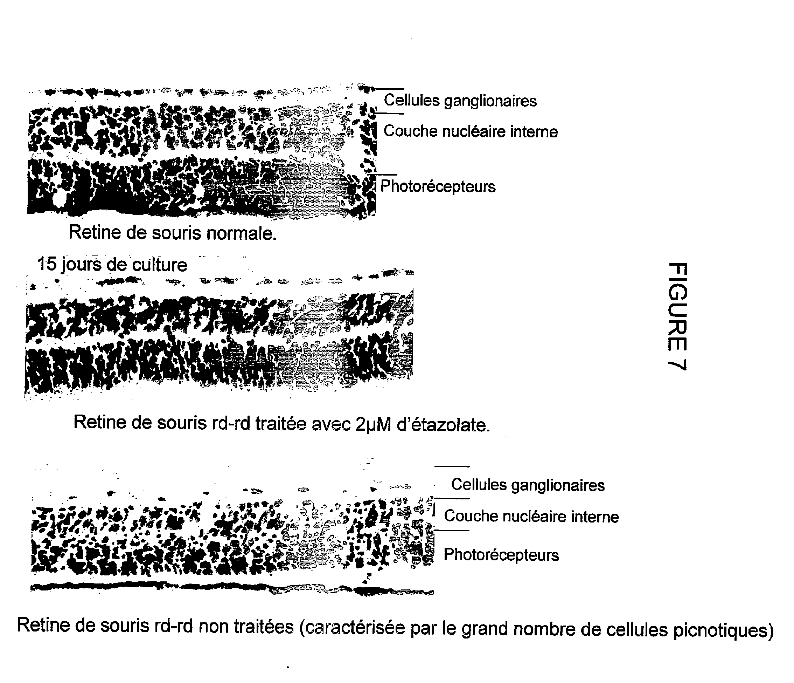

Les résultats obtenus avec 2µM d'étazolate montrent que les photorécepteurs des souris rd traités avec l'étazolate sont beaucoup mieux préservés que les photorécepteurs des souris rd-rd, non traitées. Comme le montre la figure 7, les souris rd-rd non traitées présentent les caractéristiques suivantes : un nombre élevé de cellules picnotiques et une réduction du nombre de noyau des photorécepteurs. En revanche, dans les explants traités avec l'étazolate, la couche des photorécepteurs est beaucoup mieux préservée et ressemble à celle observée sur les souris non transgéniques. Ces explants sont aussi caractérisés par une forte réduction du nombre de cellules picnotiques en comparaison avec les explants de souris rd-rd non traitées.

### Exemple 5 : Effet de l'étazolate sur le PBR.

Pour démontrer cet effet, nous réalisons des expériences sur des cellules HeLa, cellules décrites comme exprimant le PBR. Le PBR se trouve au contact entre la membrane interne et externe de la mitochondrie. Il fait parti du complexe de pore de transition, qui participe à la régulation du flux de la mitochondrie par ouverture du pore mitochondrial. Il existe des molécules ayant une forte affinité pour le PBR, notamment le Ro5-4864 et le PK11195.

En suivant la fluorescence de la rhodamine 1,2,3 spécifique aux mitochondries, nous pouvons suivre l'effet de ligands spécifiques du PBR ainsi que ceux de l'étazolate.

Les résultats ont montré que l'étazolate induisait la même perte de fluorescence que les ligands spécifiques du PBR, Ro5-4864 et PK11195.

L'étazolate apparaît donc comme un ligand du PBR qui protège les neurones contre la mort lors des phénomènes d'excitotoxicité.

### Exemple 6 : Utilisation Clinique chez l'Homme

Cet exemple décrit les conditions d'utilisation chez l'homme de l'étazolate pour le traitement de maladies neurodégénératives. Cet exemple illustre le potentiel thérapeutique de l'invention et ses conditions d'utilisation chez l'homme.

Dans cette étude, des doses uniques croissantes d'étazolate (0.5, 1, 2, 5, 10 et 20 mg) ont été administrées par voie orale sous forme de gélules dosées à 0.5 et 5 mg à des groupes différents et séquentiels de huit sujets jeunes et sains, volontaires, de sexe masculin. Cette étude a été réalisée dans un seul centre, en double aveugle et deux des huits sujets ont reçu un placebo. Les paramètres évalués ont été la tolérance clinique (apparition d'effets adverses, de signes cliniques, changement dans la pression artérielle ou la fréquence cardiaque), électrocardiographique (enregistrement de l'ECG) et biologique (hématologie et biochimie sanguine, examen urinaire) pendant les 24h suivant l'administration du produit. Un dosage plasmatique du produit a été réalisé chez chaque sujet à différents temps avant et après l'administration du produit (0,25 - 0,50 - 1,00 - 1,50 - 2,00 - 3,00 - 4,00 - 5,00 - 6,00 - 8,00 - 10,00 -12,00 et 24,00 heures). Un dosage urinaire du produit a également été réalisé à partir des urines collectées avant et après l'administration du produit (4, 4-8, 8-12 et 12-24 heures).

A l'issue de cette phase d'administration de doses croissantes, un groupe supplémentaire de six sujets reçoit à deux reprises une dose d'étazolate : à jeun et au cours d'un repas riche en graisse. L'objectif de cette seconde partie est de comparer l'évolution des taux sanguins du produit entre les deux conditions d'administration. Les paramètres évalués sont la tolérance clinique (apparition d'effets adverses, de signes cliniques, changement dans la pression artérielle ou la fréquence cardiaque), électrocardiographique (enregistrement de l'ECG) et biologique (hématologie et biochimie sanguine, examen urinaire) pendant les 24h suivant l'administration du produit. Un dosage plasmatique du produit est réalisé chez chaque sujet à différents temps avant et après l'administration du produit (0,25 - 0,50 - 1,00 - 1,50 - 2,00 - 3,00 - 4,00 - 5,00 - 6,00 - 8,00 - 10,00 - 12,00 et 24,00 heures). Un dosage urinaire du produit est également réalisé à partir des urines collectées avant et après l'administration du produit (4, 4-8, 8-12 et 12-24 heures).
Une gélule gastro-résistante est également développée pour ce produit de façon à pouvoir l'utiliser dans les études cliniques chez l'homme.

Les résultats obtenus au cours de la première phase d'étude de doses croissantes ont montré que l'étazolate était bien toléré et n'a pas entraîné d'effets secondaires. De plus, les dosages plasmatiques ont confirmé chez l'homme la bonne absorption du produit aux doses fortes.

L'ensemble de ces résultats démontre que des composés selon l'invention présentent des propriétés remarquables sur la survie des neurones, en particuliers rétiniens, et peuvent être administrés à l'homme sans effets secondaires. Ces résultats permettent donc le développement de nouvelles approches thérapeutiques efficaces des maladies neurodégénératives oculaires chez l'homme.

## Revendications

1. Utilisation de l'étazolate pour la préparation d'une composition pharmaceutique destinée au traitement d'une maladie neurodégénérative oculaire choisie parmi la dégénérescence maculaire liée à l'age, la rétinite pigmentaire et la rétinopathie.

2. Utilisation selon la revendication 1, destinée à augmenter la survie neuronale chez les patients atteints de ladite maladie neurodégénérative oculaire.

3. Utilisation selon la revendication 1, destinée à inhiber ou réduire la mort neuronale par excitotoxicité chez les patients atteints de ladite maladie neurodégénérative oculaire.

4. Utilisation selon l'une des revendications 1 à 3, **caractérisée en ce que** la maladie neurodégénérative oculaire est la rétinite pigmentaire.

5. Utilisation selon l'une des revendications 1 à 3, **caractérisée en ce que** la maladie neurodégénérative oculaire est la dégénérescence maculaire liée à l'age.

6. Utilisation selon l'une des revendications 1 à 3, **caractérisée en ce que** la maladie neurodégénérative oculaire est une rétinopathie.

7. Utilisation selon l'une des revendications 1 à 6, **caractérisée en ce que** l'étazolate est administré sous forme orale.

## Claims

1. Use of etazolate for preparing a pharmaceutical composition for the treatment of a neurodegenerative ocular disease selected from age-related macular degeneration, retinitis pigmentosa and retinopathy.

2. Use according to claim 1, to increase neuronal survival in patients suffering from said neurodegenerative ocular disease.

3. Use according to claim 1, to inhibit or reduce neuronal death due to excitotoxicity in patients suffering from said neurodegenerative ocular disease.

4. Use according to any one of claims 1 to 3, wherein the neurodegenerative ocular disease is retinitis pigmentosa.

5. Use according to any one of claims 1 to 3, wherein the neurodegenerative ocular disease is age-related macular degeneration.

6. Use according to any one of claims 1 to 3, wherein the neurodegenerative ocular disease is a retinopathy.

7. Use according to any one of claims 1 to 6, wherein etazolate is administered in oral form.

## Patentansprüche

1. Verwendung von Etazolat für die Herstellung einer pharmazeutischen Zusammensetzung zur Behandlung einer neurodegenerativen Augenerkrankung, wobei die neurodegenerative Augenerkrankung ausgewählt ist aus der altersbedingten Makuladegeneration, der Retinitis pigmentosa und der Retinopathie.

2. Verwendung nach Anspruch 1 zur Erhöhung der neuronalen Überlebensrate bei Patienten, die an der genannten neurodegenerativen Augenerkrankung leiden.

3. Verwendung nach Anspruch 1 zur Inhibierung oder Reduzierung des durch Erregungstoxizität bedingten neuronalen Tods bei Patienten, die an der genannten neurodegenerativen Augenerkrankung leiden.

4. Verwendung nach einem der Ansprüche 1-3, **dadurch gekennzeichnet, dass** die neurodegenerative Augenerkrankung die Retinitis pigmentosa ist.

5. Verwendung nach einem der Ansprüche 1-3, **dadurch gekennzeichnet, dass** die neurodegenerative Augenerkrankung die altersbedingte Makuladegeneration ist.

6. Verwendung nach einem der Ansprüche 1-3, **dadurch gekennzeichnet, dass** die neurodegenerative Augenerkrankung eine Retinopathie ist.

7. Verwendung nach einem der Ansprüche 1-6, **dadurch gekennzeichnet, dass** Etazolat oral verabreicht wird.


### SEQUENCE LISTING

<110> EXONHIT THERAPEUTICS
<120> Méthodes et compositions pour le traitement de pathologies dégénératives oculaires
<130> B0189WO
<150> FR 03 02021
   <151> 2003-02-19
<160> 6
<170> PatentIn version 3.1
<210> 1
   <211> 20
   <212> DNA
   <213> artificial sequence
<220>
   <223> amorce
   <400> 1
   gccaggccgt gaagcaaata 20
<210> 2
   <211> 18
   <212> DNA
   <213> artificiel sequence
<220>
   <223> amorce
   <400> 2
   tcaaagacgc gaaaacat 18
<210> 3
   <211> 21
   <212> DNA
   <213> artificial sequence
<220>
   <223> amorce
   <400> 3
   ccgcgtcagt gcctttgcta t 21
<210> 4
   <211> 23
   <212> DNA
   <213> artificial sequence
<220>
   <223> amorce
   <400> 4
   cgctgtcgga tgcttttatt cac 23
<210> 5
   <211> 19
   <212> DNA
   <213> artificial sequence
<220>
   <223> amorce
   <400> 5
   tcgctttctg gagggtgtc 19
<210> 6
   <211> 18
   <212> DNA
   <213> artificial sequence
<220>
   <223> amorce
   <400> 6
   ccgcaggggc agcagtgg 18